# EUROPÄISCHE PATENTSCHRIFT

(11) **EP 3 116 308 B1**
(45) Veröffentlichungstag und Bekanntmachung des Hinweises auf die Patenterteilung: **07.04.2021**
(21) Anmeldenummer: 15709194.3
(22) Anmeldetag: 12.03.2015
(51) Int. Cl.: A01M 1/02, A01N 59/04, A01N 63/00

(54) **LOCKSYSTEM FÜR FLUGINSEKTEN UND SPINNENTIERE**
SYSTEM FOR ATTRACTING FLYING INSECTS AND ARACHNIDS
SYSTÈME D'APPÂT POUR LES INSECTES VOLANTS ET LES ARACHNIDES

(30) Priorität: 12.03.2014 DE 102014103268
(43) Veröffentlichungstag der Anmeldung: 18.01.2017
(73) Patentinhaber: BIOCARE Gesellschaft für biologische Schutzmittel mbH, 37574 Einbeck (DE)
(72) Erfinder: PATEL, Anant, 33604 Bielefeld (DE)
(74) Vertreter: Gramm, Lins & Partner Patent- und Rechtsanwälte PartGmbB
(86) Internationale Anmeldenummer: PCT/EP2015/055133
(87) Internationale Veröffentlichungsnummer: WO 2015/136023

(56) Entgegenhaltungen:
- WO-A1-01/32013
- WO-A1-2014/041112
- US-A1- 2013 291 427
- US-B1- 6 978 572

## Beschreibung

Die vorliegende Erfindung richtet sich auf die Verwendung eines Systems bzw. einer Vorrichtung zum Locken und gegebenenfalls Töten von Fluginsekten und Spinnentieren in einer gasförmigen Umgebung. Das erfindungsgemäße zu verwendende System bzw. die erfindungsgemäße zu verwendende Vorrichtung umfasst CO₂-freisetzende Mikroorganismen, für diese Mikroorganismen spezifische Nährstoffe und biologisch abbaubare Biopolymere, wobei dieses System derart ausgebildet ist, dass diese biologisch abbaubaren Biopolymere die weiteren, insbesondere die genannten Bestandteile ein- oder umschließt gemäß Anspruch 1. Das System oder die Vorrichtung kann derart ausgebildet sein, dass es CO₂ über einen Zeitraum von mehr als 20 Tagen in einer gasförmigen Umgebung freisetzt, zum Locken der Fluginsekten und Spinnentiere.

### Stand der Technik

Für im Boden lebende Organismen ist die Verwendung von Kohlenstoffdioxid (CO₂) als Lockstoff beschrieben. Eine Vielzahl von Bodenschädlingen nutzen CO₂ und dessen Konzentration zur Lokalisierung der Wirte. Ein häufig beschriebenes Beispiel hierfür ist der westliche Maiswurzelbohrer (*Diabrotica virgifera virgifera*), deren Larven CO₂ zur Lokalisierung von Wurzeln von lebenden Maispflanzen verwendet, um diese dann als Nahrung zu nutzen. Dabei bewegen sich die Larven in Richtung des aufsteigenden CO₂ Gradienten, da die Maispflanze CO₂ freisetzt und somit die Konzentration an CO₂ an oder in der Nähe der Pflanzen bzw. Pflanzenwurzeln größer ist als in weiter entfernten Gebieten.

Die Zerstörung der Wurzeln und der damit einhergehende physiologische Stress der Pflanzen durch das Fressverhalten der Larven führen zu hohen Verlusten bei Nutzpflanzen.

Für solche Bodenschädlinge sind verschiedene Locksysteme und Lockfallen beschrieben. Neben verschiedenen üblichen chemischen oder biologischen Pestiziden wurde versucht, die Lokalisierung der Wirte durch diesen Schädling, die, wie gesagt, durch einen ansteigenden CO₂ Gradienten erfolgt, zu stören. Hierzu werden von Bernklau E. A. et al. verschiedene Möglichkeiten beschrieben, siehe z. B. Bernklau E. A. et al., Journal of Economic Entomology, 97(2), 330-339, 2004. In diesem Dokument werden Verfahren und Vorrichtungen zum Anlocken von Schädlingen genannt. Dabei werden Körner aus Bäckerhefe und entsprechenden Nährstoffen hierfür, sowie mit organischem Substrat beschrieben, die dann als CO₂ Quelle dienen, um die Larven des westlichen Maiswurzelbohrers von den Wurzeln der Maispflanzen fernzuhalten. Ähnliche Verfahren werden von Bernklau et al. in den Patentanmeldungen WO 01/32013 A1 und US 6978572 B1 offenbart. Darin werden Formulierung beschrieben, die unter anderem aus einem CO₂-freisetzenden Mittel, wie Hefen, Nährstoffe hierfür und Polymer umfassen. Dabei werden Körnchen hergestellt, wobei das Polymer als Matrix dient. Es wird insbesondere beschrieben, dass diese aus dem Polymer gebildete Matrix eine schwammartige Struktur bzw. poröse Struktur aufweist. Eine solche Struktur soll ein verlängertes Freisetzen von CO₂ erlauben.

Alle beschriebenen Vorrichtungen und Verfahren haben aber das Problem, dass sie den CO₂ Gradienten und damit die Wirksamkeit dieses Systems nur für im und/oder auf dem Boden lebende Schädlingen und auch nur für einen kurzen Zeitraum ermöglichen. Für die bodenständigen Verfahren sind Zeiträume von maximal 2 Wochen beschrieben.

Die nachveröffentlichte WO 2014/041112 A1 beschreibt Locksysteme insbesondere zur Verwendung im Boden.

Für luftbasierte Systeme, d.h. Locksysteme, die nicht im Boden sondern in gasgefüllten Räumen an der Luft wirksam sind und insbesondere solche Systeme gerichtet zum Locken von Fluginsekten und Spinnentieren, gibt es keine auf CO₂ basierende kommerziellen Techniken und Verfahren. Kürzlich wurde von Smallegange R.C. et al., Malaria Journal, 2010, 9, 292 beschreiben, dass eine Mischung aus Hefe, Zuckerlösung und Wasser in Fallen für Mücken, dieses Mücken anlocken kann. Allerdings zeigte sich, dass die Ausbildung eines CO₂ Gradienten nur kurzzeitig erfolgt. Bereits nach 34 Stunden konnte ein deutlicher Abfall der CO₂ Produktion durch die Hefen beobachtet werden. Außerdem ist das dort beschriebene System schwierig zu handhaben und zeigt eine große Kontaminationsgefahr auf.

Ziel der vorliegenden Anmeldung ist die Bereitstellung von Systemen, die ein Locken und gegebenenfalls Immobilisieren und letztendlich Töten von Fluginsekten und Spinnentieren erlaubt, weiterhin ist die Bereitstellung von entsprechenden Verfahren zur Bekämpfung dieser Fluginsekten und Spinnentiere sowie die Verwendung von Systemen zur Bekämpfung dieser Tiere. Die erfindungsgemäß zu verwendenden Systeme und Vorrichtungen erlauben die Bekämpfung von Luftschädlingen, insbesondere von solchen Fluginsekten und Spinnentieren, die potentiell Parasiten übertragen. Vorliegend wird unter Parasiten dabei sowohl Mikro- als auch Makroparasiten, also sowohl Mikroorganismen einschließlich eukaryotische und prokaryotische Mikroorganismen, wie Bakterien, Protozoen und Pilze aber auch Viren etc. verstanden.

Insbesondere geeignet sind die erfindungsgemäß zu verwendenden Systeme oder Vorrichtungen zur Bekämpfung von Parasiten übertragenden Fluginsekten, z. B. Stechmücken, Tse-Tse-Fliegen usw. aber auch von Spinnentieren, wie Zecken und Milben etc.

### Beschreibung der Erfindung

Erfindungsgemäß wird die Verwendung eines Systems bzw. eine Vorrichtung zum Locken und gegebenenfalls Immobilisieren und letztendlich gegebenenfalls Töten von Fluginsekten (Pterygota) und Spinnentiere (Arachnida) in einer gasförmigen Umgebung umfassend CO₂ freisetzende Mikroorganismen, für diese Mikroorganismen spezifische Nährstoffe und ein biologisch abbaubares Biopolymer gemäß Anspruch 1 bereitgestellt. Das biologisch abbaubare Biopolymer ist dabei derart ausgebildet, dass es die weiteren, insbesondere oben genannten Bestandteile ein- oder umschließt, z. B. in Form einer Kapsel, eines Pellets, eines Granulats, von Partikeln, Streifen, Schichten, Fasern, usw. In einer Ausführungsform setzt das System oder diese Vorrichtung CO₂ in einer gasförmigen Umgebung über eine Zeit von mehr als 20 Tagen frei, zum Locken der Fluginsekten und Spinnentieren. In einer Ausführungsform kann das Biopolymer dabei die weiteren Bestandteile vollständig umschließen.

Es ist möglich, dass die Freisetzung des CO₂ einen Zeitraum von mehr als 20, wie mehr als 25 Tagen, wie über 28 Tage und länger, z. B. auf 35 Tage und mehr erfolgt.

Vorliegend werden die Ausdrücke "System" und "Vorrichtung" synonym eingesetzt, soweit nicht anders ausgeführt.

Unter dem Ausdruck "über einen Zeitraum von mehr als 20 Tagen" wird vorliegend verstanden, dass die CO₂ freisetzenden Mikroorganismen im erfindungsgemäßen System über einen Zeitraum von mehr als 20 Tagen aktiv CO₂ in einer gasförmigen Umgebung freisetzen. Das heißt, zumindest Teile der Mikroorganismen liegen im System über diesem genannten Zeitraum lebend vor und setzen das CO₂ aktiv frei. Es ist insbesondere darunter zu verstehen, dass die CO₂ Konzentration in unmittelbarer Umgebung des Systems oberhalb (über) der CO₂ Konzentration eines von dem System entfernter liegenden Bereichs liegt, sodass ein CO₂ Gradient in Richtung des Systems ausgebildet wird.

Überraschend zeigt es sich, dass die Ausbildung eines CO₂ Gradienten auch für Systeme und Vorrichtungen gilt, die in der Luft vorliegen, d.h. in einer gasförmigen Umgebung.

Erfindungsgemäß handelt es sich bei dem System bzw. der Vorrichtung um solche gebildet aus Kapseln, gegebenenfalls auch Pellets oder Granulate, wobei zumindest die Kapselhülle bzw. die Kapselmatrix durch ein oder mehrere Biopolymere ausgebildet ist. Hierbei kann es sich um solche handeln, die Biopolymer vollständig enthalten, z. B. in Form von Pellets. Die Pellets oder Kapseln können dabei in einer Ausführungsform im Wesentlichen von dem Biopolymer vollständig umschlossen sein. Dass heißt, das biologisch abbaubare Biopolymer bildet ein System aus, dass die weiteren genannten Bestandteile, wie Mikroorganismen und Nährstoffe für die Mikroorganismen, ein- oder umschließt. Weiterhin kann es sich um Granulate handeln, wobei bei diesen Granulaten, die ein oder mehrere Biopolymere aufweisen, die weiteren Bestandteile ebenfalls bevorzugt umschließen so dass diese nicht aus dem System oder der Vorrichtung heraustreten können, oder als vernetzende und stabilisierende Matrix dienen. Weiterhin sind Ausführungsformen des erfindungsgemäßen Systems möglich, bei denen die Biopolymere die Partikel, wie Kapseln, Pellets oder Granulate aus den weiteren Bestandteilen durchziehen und somit die notwendige Stabilität des Systems liefern. Die Systeme und Vorrichtungen können derart ausgebildet sein, dass gasförmige Substanzen, wie CO2, freigesetzt werden, während solide und flüssige Substanzern im System oder der Vorrichtung verbleiben.

Weitere Formen schließen ein: Streifen, Fasern oder Beschichtungen. Beschichtungen können z.B. auf verschiedene Grundkörper aufgebracht sein, je nach Anwendung z.B. auf Gitter, Halsbänder, z.B. Zeckenhalsbänder aber auch allgemein in Form von Partikeln, etc. Dem Fachmann sind geeignete Strukturen zum Beschichten bekannt.

Die Systeme oder Vorrichtungen in Form von entsprechenden Partikeln, wie Kapseln, Pellets oder Granulate können weiterhin an der Oberfläche Beschichtungen oder allgemein Einlagerungen von Insektiziden und/oder Akariziden aufweisen, die die anzulockenden Fluginsekten und Spinnentiere immobilisieren und gegebenenfalls töten können. Die Oberfläche der erfindungsgemäßen Systeme und Vorrichtungen können auch weitere Bestandteile zum Immobilisieren der angelockten Fluginsekten und Spinnentiere aufweisen, wie z. B. adhäsive Bestandteile, um diese Fluginsekten und Spinnentiere zu immobilisieren.

Unter den Ausdrücken "Insektizide" und "Akarizide" werden dabei vorliegend Wirkstoffe verstanden, die die genannten Fluginsekten (Insektizide) und/oder Spinnentiere (Akarizide) lähmen und gegebenenfalls töten können.

In einer Ausführungsform liegen somit im erfindungsgemäß zu verwendenden System bzw. in der erfindungsgemäß zu verwendenden Vorrichtung weiterhin Insektizide und/oder Akarizide, insbesondere solche ausgewählt aus der Gruppe der chemischen Insektizide, chemischen Akarizide, Pflanzenextrakte, entomopathogene Mikroorganismen vor. Diese Insektizide bzw. Akarizide wirken z. B. spezifisch gegen bestimmte Fluginsekten, wie Mücken, Fliegen usw. oder Spinnentiere, wie Zecken usw., die Parasiten insbesondere auf den Menschen aber auch auf Tiere übertragen können.

Bei den im System vorliegenden CO₂ freisetzenden Mikroorganismen handelt es sich insbesondere um Pilze oder Bakterien. Bevorzugt sind die Mikroorganismen Hefen einschließlich kommerziell erhältlichen Hefen, wie Bäckerhefe, aber auch Resthefe aus der Bier-, Wein- und Bioethanol-Produktion. Es können ein oder mehrere verschiedene Mikroorganismen vorliegen, die entweder ebenfalls CO₂ freisetzen und/oder Hilfsfunktionen aufweisen.

Weiterhin kann ein mindestens zweiter Mikroorganismus, auch als Hilfsorganismus bezeichnet, einer mit insektizider bzw. akarizider Wirkung sein. Dem Fachmann sind geeignete Mikroorganismen bekannt. Bei diesen kann es sich z. B. um einen zweiten Mikroorganismus handeln, der entomopathogen wirkt. Unter "entomopathogen" wird vorliegend verstanden, dass der Mikroorganismus die Fähigkeit besitzt. Insekten bzw. Spinnentiere zu befallen, zu töten, zu immobilisieren oder anderweitig unschädlich zu machen.

Es liegt ein mindestens zweiter Mikroorganismus vor, der die Nährstoffversorgung der CO₂ freisetzenden Mikroorganismen unterstützt, zum Beispiel nährstoff-mobilisierende Mikroorganismen. Diese Mikroorganismen stellen für die CO₂ freisetzenden Mikroorganismen eine C-Quelle und/oder N-Quelle bereit.

Ein Beispiel hierfür stellt der Pilz *Beauveria bassiana* dar. Dieser Pilz ist bekannt dafür, dass es parasitär auf verschiedene arthropode Spezien wirkt. Weiterhin ist er als biologisches Insektizid bekannt. Es zeigte sich, dass aber auch als Alternative hierzu ein Pilz aus der Gattung der Metarhizien, wie *M. anisopliae,* die im System vorliegenden Nährstoffe, in einer Weise umsetzen kann, dass diese durch die für die CO₂ Produktion verantwortlichen Mikroorganismen besser verstoffwechselt werden können.

Vorliegend wird unter zweiter Mikroorganismus oder Hilfsorganismus sowohl der gesamte, lebende Mikroorganismus als auch einzelne Enzyme oder Mischungen von Enzymen verstanden.

Zum Beispiel ermöglichen die genannten Pilze *B*. *bassiana* und *M. anisopliae* ein Abbau von Stärke, die als speziellen Nährstoff für den Mikroorganismus in dem System vorliegt. Diese genannten Pilze weisen eine Amylase-Aktivität auf, und können so die Stärke entsprechend abbauen. In einer Ausführungsform liegt daher ein zweiter Mikroorganismus, auch als Hilfsorganismus bezeichnet, vor, der eine Amylase-Aktivität aufweist und Nährstoffe, z. B. in Form von Stärke abbauen kann, damit diese den CO₂ Mikroorganismus zugänglich sind. *B*. *bassiana* oder *M. anisopliae* können vorliegend somit zwei Funktionen erfüllen, einerseits weisen sie eine insektizide oder akarizide Wirkung auf. Andererseits erlauben sie die Umsetzung von Nährstoffen, um diese für die CO₂ Freisetzung verantwortlichen Mikroorganismen besser verstoffwechselbar zu machen, z. B. aufgrund der genannten Amylase-Aktivität in diesen Mikroorganismen.

Weiterhin ist es möglich, dass als Bestandteile des erfindungsgemäß zu verwendenden Systems oder der erfindungsgemäß zu verwendenden Vorrichtung nicht die gesamten Hilfsorganismen vorliegen sondern einzelne Enzyme, wie z.B. Amylasen oder andere Glykosidasen, Proteasen etc.

Bei den für diese Mikroorganismen spezifischen Nährstoffen handelt es sich z. B. um solche ausgewählt aus Getreidemehl, Maismehl, Maisprotein und anderen Maisbestandteilen, Stärke, Kürbismehl, Kartoffelmehl. Daneben können aber auch Roh- und Reststoffe aus der Agrar- und Lebensmittelindustrie eingesetzt werden. Sollte zum Beispiel Cellulose verwendet werden, werden als Hilfsorganismen oder Hilfsstoffe Cellulase oder Cellulase enthaltende Mikroorganismen eingesetzt.

Dem Fachmann sind im Übrigen geeignete Nährstoffe für die in dem System vorliegenden Mikroorganismen bekannt. Diese Nährstoffe sind dabei nicht nur solche, die zu CO₂ umgesetzt werden, sondern auch solche, die die Mikroorganismen zum Überleben benötigen, einschließlich N-Quelle etc.

Bei den Fluginsekten handelt es sich insbesondere um solche aus Diptera, Neoptera, Hymenoptera, Coleoptera und Heteroptera, insbesondere Fliegen, Mücken, Wanzen und Termiten. Das System eignet sich insbesondere zum Locken von mit Parasiten befallenen oder möglicherweise befallenen Fluginsekten, wie Tse-Tse-Fliegen, Stechmücken. In einer bevorzugten Ausführungsform handelt es sich bei den Spinnentieren um Milben, wie Zecken.

Wie bereits erwähnt, ist in einer Ausführungsform das System bzw. die Vorrichtung eines, des weiteren ein Insektizid und/oder Akarizid aufweist. Dieses Insektizid und/oder Akarizid kann ein chemisches Insektizid und/oder chemisches Akarizid sein oder eines auf Pflanzenbasis, aber auch solche wie die genannten entomopathogenen Pilze usw.

Es zeigte sich, dass das erfindungsgemäß verwendete System bzw. die erfindungsgemäß verwendete Vorrichtung, z. B. in Form von Kapseln, wie Pellets, die Hefe, Maismehl oder andere Stärkequellen und Hilfsorganismen, wie *B*. *bassiana* oder *M. anisopliae* enthalten, die gewünschten Eigenschaften der Ausbildung einer entsprechenden CO₂ Konzentration zum Anlocken von Fluginsekten und Spinnentieren in gasförmiger Umgebung, z. B. in der Luft erlauben.

Entsprechend können die erfindungsgemäß verwendetenn Systeme oder Vorrichtungen zum Locken und gegebenenfalls Immobilisieren und gegebenenfalls Töten der Fluginsekten und Spinnentiere eingesetzt werden. Diese Systeme können z. B. derart angeordnet sein, dass sie in der Nähe von Fenstern und Türen vorliegen, um in abgeschlossene Räume oder Gebäude eintretende Fluginsekten oder Spinnentiere abzufangen. Zum Beispiel können diese Systeme oder Vorrichtungen zum Locken dieser Fluginsekten und Spinnentiere an Fliegengittern und Netzen angeordnet oder integriert sein, um entsprechend die Fluginsekten und/oder Spinnentiere zu locken und zu immobilisieren. Solche Systeme und Vorrichtungen sind insbesondere in Regionen geeignet, wo Menschen der Gefahr ausgesetzt sind, mit Parasiten befallenen oder potenziell befallenen Fluginsekten oder Spinnentieren in Kontakt zu kommen und von diesen gebissen oder gestochen zu werden. Das vorliegende System oder die vorliegende Vorrichtung kann einfach und günstig hergestellt und eingesetzt werden. Damit eignet sich dieses System besonders für Regionen mit geringem Einkommen, wie Regionen in Afrika, Asien usw. Das erfindungsgemäße System kann z. B. zur Bekämpfung von Malaria übertragenden Tse-Tse-Fliegen eingesetzt werden. Die erfindungsgemäßen Systeme sind dabei in bekannten Locksystemen einsetzbar. Aufgrund der ein- oder umschließenden Biopolymers ist die Gefahr gering, dass durch in Kontakt kommen mit dem System eine Gefahr für den Menschen oder Tieren besteht und die Belastung des Menschen oder Tieren durch Gefahrstoffe, wie Insektizide, ist verringert.

Das System kann weiterhin weitere Bestandteile aufweisen, wie Füllstoffe, Trocknungshilfsmittel, Glukose, Saccharose, weitere Lockstoffe wie Isolate aus menschlichen Körperschweiss, Vogelfedern, Pferdehaaren oder andere bekannte chemische Lockstoffe für die jeweiligen Fluginsekten und/oder Spinnentiere. Weiterhin können auch Cellulose, Lignin und/oder Quellungshilfen vorhanden sein. Dem Fachmann sind geeignete weitere Bestandteile bekannt, wobei diese nicht derart mit dem System vorliegenden Mikroorganismen interagieren, dass diese in dem System vorliegenden Mikroorganismen getötet oder anderweitig in ihrer Funktion beschränkt werden.

Die erfindungsgemäß verwendbaren Systeme zeichnen sich dadurch aus, dass sie ein oder mehrere biologisch abbaubare Biopolymere aufweisen. Diese Biopolymere ein- oder umschließen dabei die Mikroorganismen und Nährstoffe und bilden z.B. eine Kapselhülle aus, wenn das System vollständig umschlossen als Kapsel vorliegt. Diese Kapselhülle kann dabei derart ausgebildet sein, dass sie ein Austausch von Nährstoffen und insbesondere Austreten von CO₂ ermöglicht, d.h., dass gasförmige Bestandteile durchtreten können, während flüssige oder feste Bestandteile nicht die Kapselhülle durchtreten können.

In einer alternativen Ausführungsform ist das erfindungsgemäß verwendbare System eines, bei dem das Biopolymer eine Matrix ausbildet. Dabei werden die übrigen Bestandteile in die Matrix eingeschlossen oder die Matrix umschließt die Bestandteile, wobei das Umschließen ein teilweises Umschließen sein kann oder ein vollständiges Umschließen. Dabei sind diese so ausgebildet, dass die Biopolymere in einer Ausführungsform die weiteren Bestandteile bevorzugt im Wesentlichen, wie vollständig, umschließen.

Bei dem biologisch abbaubaren Biopolymer handelt es sich insbesondere um solche ausgewählt aus Alginat, Carrageenan, Cellulose, Hemicellulose, Stärke, Chitin, Chitosan, Pektinat, Guar Gum, Acacia Gum, poly-D, L-Milchsäure, Gelatine, poly-Aminosäuren, Lignin und Derivaten sowie Mischungen hiervon. Dem Fachmann sind entsprechend geeignete Biopolymere bekannt. Es kann z. B. Alginat oder einer Alginat-Gelatine-Mischung eingesetzt werden.

Die erfindungsgemäß verwendbaren Systeme und Vorrichtungen können aber auch solche sein, bei denen die kapselausbildenden Polymere zumindest teilweise nicht-abbaubare Polymere sind. Die Polymere müssen allerdings den Anforderungen genügen, dass sie durchlässig für CO₂ und gegebenenfalls andere gasförmige Bestandteile sind, damit die im System vorliegenden Mikroorganismen über einen längeren Zeitraum CO₂ freisetzen können, um so die genannten Fluginsekten und Spinnentiere anzulocken und gegebenenfalls zu immobilisieren.

Die vorliegende Anmeldung richtet sich auf die Verwendung eines Systems bzw. einer Vorrichtung zum Locken und gegebenenfalls Immobilisieren und gegebenenfalls letztendlich Töten von Fluginsekten (Pterygota) und/oder Spinnentieren (Arachnida), insbesondere zum Anlocken und Töten von potentiell Parasiten-übertragenden Fluginsekten und Spinnentieren.

Besonders bevorzugt wird das erfindungsgemäß verwendbare System bzw. die erfindungsgemäß verwendbare Vorrichtung eingesetzt, um in Räumen, einschließlich Zelten, oder allgemein in abgetrennten Bereichen potenziell Parasiten übertragende Fluginsekten und Spinnentiere zu bekämpfen. Zum Beispiel eignet sich das vorliegendeVerfahren zur Verwendung bei der Bekämpfung von Fluginsekten, wie Tse-Tse-Fliegen, Stechmücken, aber auch Zecken usw. Es ist aber auch möglich, das erfindungsgemäß verwendbare System bzw. die erfindungsgemäß verwendbare Vorrichtung außerhalb von Räumen, d. h. in der Natur, wie z. B. in Wäldern einzusetzen, um Fluginsekten oder allgemein flugfähige Schädlinge zu bekämpfen. Das System eignet sich zur Bekämpfung von potentiell Parasiten übertragenden Fluginsekten und Spinnentieren, wobei die Parasiten u. a. solche sind, die Tiere, wie Säugetiere infizieren und befallen können. Beispielhaft seien hier genannt Nutztiere und hier insbesondere landwirtschaftliche Nutztiere einschließlich Geflügel und Rinder, Ziegen, Schafe etc.

Das System ist bevorzugt eines, das weiterhin die Fluginsekten und/oder Spinnentiere immobilisiert, auch als "attract and trap" bekannt, und letztendlich tötet, allgemein als "attract and kill"-Ansatz bekannt. Dabei können die Systeme neben dem CO₂ freisetzenden Mikroorganismus weitere Mikroorganismen enthalten, die eine insektizide bzw. akarizide Wirkung besitzen und/oder die Versorgung der CO₂ freisetzenden Mikroorganismen mit Nährstoffen unterstützen, in dem sie diese Nährstoffe abbauen, z. B. aufgrund einer vorliegenden Amylase-Aktivität. Weiterhin können bekannte Stoffe vorliegen, die ein Töten der angelockten Insekten und Spinnentiere bewirken.

Die Systeme können weiterhin entsprechend geeignete pflanzliche oder chemische Wirkstoffe zum Abtöten der Fluginsekten und Spinnentiere aufweisen.

Schließlich werden Verfahren zum Anlocken und gegebenenfalls Immobilisieren und Töten dieser Fluginsekten und Spinnentiere bereitgestellt. Dieses Verfahren umfasst dabei das entsprechende Bereitstellen eines erfindungsgemäß zu verwendenden Systems oder einer erfindungsgemäß zu verwendenden Vorrichtung und Positionieren dieser derart dass die Fluginsekten und/oder Spinnentiere angelockt werden. Entsprechendes Positionieren umfasst ein Positionieren in Eintrittsbereichen von Gebäuden oder abgetrennten Bereichen. Dabei können diese Systeme oder Vorrichtungen mit entsprechenden Einrichtungen, z. B. Fliegengitter oder Mückennetze etc. bereitgestellt werden, um die Fluginsekten und Spinnentiere anzulocken und gegebenenfalls zu töten.

Dem Fachmann sind geeignete Möglichkeiten und Einrichtungen zur Durchführung des Verfahrens bekannt.

Das vorliegende Verfahren ist insbesondere eines, dass sich dadurch auszeichnet, dass durch eine erhöhte CO₂ Konzentration in einer gasförmigen Umgebung im und in der Nähe des erfindungsgemäß zu verwendenden Systems oder Vorrichtung die Fluginsekten und Spinnentiere in Richtung dieses System und Vorrichtung gelockt werden um dort immobilisiert und gegebenenfalls getötet zu werden. Das Verfahren eignet sich insbesondere diese Fluginsekten und Spinnentiere über einen langen Zeitraum anzulocken und zu töten. In einer Ausführungsform ist dieser Zeitraum mindestens einer von 20 Tagen, bevorzugt mindestens 25 Tage, wie mindestens 30 Tage.

Besonders geeignete Ausführungsformen sind solche, bei denen es sich um Kapseln oder Pellets handelt, umfassend ein Biopolymer oder eine Biopolymermischung, mindestens ein CO₂ freisetzenden Mikroorganismus, wie Hefe, Nährstoffe, wie stärkehaltige Nährstoffe, Hilfsmikroorganismen, wie z. B. *B*. *bassiana* oder *M. anisopliae,* oder Enzyme und gegebenenfalls weitere Kill-Komponenten.

Die erfindungsgemäß verwendbaren Systeme und Vorrichtungen können weitere Lockstoffe aufweisen, die neben oder zusätzlich zu CO₂ die Fluginsekten und Spinnentiere anlocken. Insbesondere sind diese Lockstoffe, die auf geringe Entfernung die Fluginsekten und/oder Spinnentiere in Richtung des Systems locken, damit sie dort gegebenenfalls getötet werden können.

Im Folgenden wird die erfindungsgemäße Verwendung der Systeme näher beschrieben, ohne auf diese begrenzt zu sein.

### Beispiele:

### Beispiel 1

### Formulierung des erfindungsgemäß verwendbaren Systems

*S*. *cervisiae* wird in vorbestimmter Menge entweder als angezogener Stamm oder als kommerziell erhältlicher Bäckerhefemix gegebenenfalls mit den weiteren Bestandteilen, wie Stärke und/oder Hilfsorganismen, wie *Beauveria bassiana* oder *M. anisopliae,* oder Enzymen, wie Amylase, in 2%igem Natriumalginat suspendiert und mit Hilfe einer herkömmlichen Verkapselungsvorrichtung in eine 2%ige CaCl₂ Lösung eingetropft und 20 min nachvernetzt. Die Kapseln wurden mit einem Durchmesser von durchschnittlich 2,7 mm hergestellt.

### Beispiel 2

### Verwendung des erfindungsgemäß verwendbaren Systems zum Anlocken und gegebenenfalls Immobilisieren von Zecken

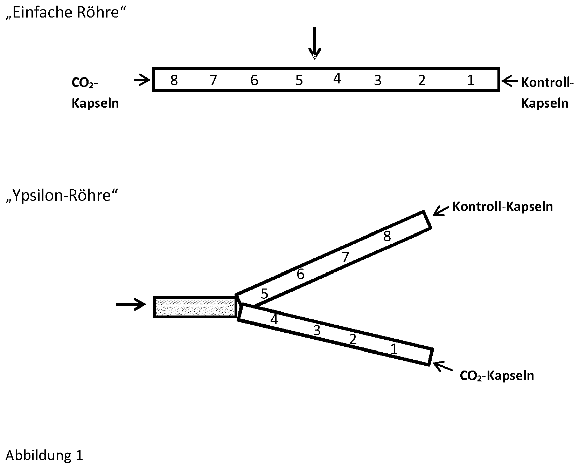

Zecken (*Ixodes ricinus*) wurden in eine einfache Röhre oder eine Y-förmige Röhre an der Position des Pfeils eingebracht, Fig.1. Die Zahlen geben dabei die Probenahmepunkte der CO₂ Messung an.

Es wurde 24 h gewartet und anschließend bestimmt, in welchen Sektor sich die Zecken aufhalten. Die Kapseln wurden dabei jeweils am Rohrende platziert. In der Tabelle 1 und der Tabelle 2 sind die Ergebnisse von 2 Experimenten dargestellt. Weiterhin ist der CO₂-Gradient von der Pos 8 bis 1 bzw. Pos 8 bis 5 (Y-Rohr) dargestellt.

**Tabelle 1 einfaches Rohr**

| Versuch | Anzahl Zecken | CO2 Seite | Kontrollseite | Kreuzung | Gradient |
|---|---|---|---|---|---|
| 1 | 12 | 9 | 2 | 1 | 890-740 ppm |
| 2 | 11 | 8 | 2 | 1 | 840-800 ppm |

**Tabelle 2 Y-Rohr**

| Versuch | Anzahl Zecken | CO2 Seite | Kontrollseite | Kreuzung | Anfangsrohr | Gradient |
|---|---|---|---|---|---|---|
| 1 | 19 | 12 | 2 | 2 | 3 | 890-740 ppm |
| 2 | 19 | 8 | 2 | 1 | 8 | 620-510 ppm |

Es ist deutlich zu erkennen, dass sich die Zecken in Richtung der CO₂-freisetzenden Kapseln bewegen, in Richtung des aufsteigenden CO₂-Gradienten.

Es wurde weiterhin ein Kill-Versuch durch den Nutzpilz *M. anisopliae* als Kill-Komponente durchgeführt. Dazu wurden die Zecken-Nymphen für 30 s in eine Pilzsporensuspension (1%) bzw. als Kontrolle in Wasser eingetaucht. Während die Zecken nach der Kontrollbehandlung noch nach mehreren Tagen vital waren, waren bei den mit der Pilzsporensuspension behandelten Zecken 50 % verstorben, der Rest zeigt nur geringe Vitalität. Bei einem Auswachsen der toten Zecken auf einer Selektivagarplatte wuchsen Pilze aus.

Somit ist ein relativ geringer CO₂-Gradient ausreichend, um Zecken anzulocken. Der Pilz *M. anisopliae* ist als kill-Komponente geeignet neben seiner Eigenschaft, in der Kapseln vorliegende Nährstoffe in Form von Stärke aufgrund seiner Amylase umzuwandeln, um als Nährstoff für die Hefe zu dienen.

### Beispiel 3

### Verwendung des erfindungsgemäß verwendbaren Systems zum Anlocken und gegebenenfalls Immobilisieren der gemeinen Stechmücke (Culex pipiens)

Adulte Mücken wurden in einer einfachen Röhre ähnlich der in Fig. 1 gezeigten eingebracht. Die CO₂-freisetzenden Kapseln und die Kontrollkapseln wurden entsprechend an den Enden platziert. Bei den CO₂-freisetzenden Kapseln handelt es sich um solche mit Bäckerhefe, 16,7 Gew.-%, Stärke 20 Gew.-%, Amylase 0,5 U/g Kapsel.

Luft wurde von den Enden der Röhre in Richtung Mitte gefächert. In der Tabelle 3 sind die Ergebnisse eines Versuchs nach einem Tag dargestellt.

**Tabelle 3**

| Versuch | Anzahl Mücken | CO₂ Seite | Kontrollseite | Kreuzung |
|---|---|---|---|---|
| 1 | 11 | 9 | 1 | 1 |

Es zeigte sich, dass auch die Mücken bereits bei einem geringen CO₂-Gradienten sich in Richtung des ansteigenden CO₂ Gradienten bewegen und damit erfolgreich angelockt werden können.

## Patentansprüche

1. Verwendung eines Systems zum Locken und gegebenenfalls Töten von Fluginsekten und/oder Spinnentieren in einer gasförmigen Umgebung umfassend i) CO₂-freisetzende Mikroorganismen, ii) für diese Mikroorganismen spezifische Nährstoffe, iii) Hilfsorganismen und/oder Enzyme, die die Nährstoffversorgung der CO₂-freisetzenden Mikroorganismen unterstützen, und iv) ein biologisch abbaubares Biopolymer, wobei dieses biologisch abbaubare Biopolymer die weiteren Bestandteile ein- oder umschließt.

2. Verwendung eines Systems zum Anlocken von Fluginsekten und/oder Spinnentieren nach Anspruch 1 zum Locken der Fluginsekten und Spinnentiere in einer gasförmigen Umgebung, wobei dieses System derart ausgebildet ist, dass die Freisetzung von CO₂ über eine Zeit von mehr als 20 Tagen erfolgt.

3. Verwendung eines Systems zum Anlocken von Fluginsekten und/oder Spinnentieren in einer gasförmigen Umgebung nach Anspruch 1 oder 2, wobei die CO₂-freisetzenden Mikroorganismen ausgewählt sind aus Pilzen oder Bakterien, wie Hefe, insbesondere Bäckerhefe.

4. Verwendung eines Systems zum Anlocken von Fluginsekten und/oder Spinnentieren in einer gasförmigen Umgebung nach einem der vorherigen Ansprüche, wobei die Nährstoffe ausgewählt sind aus Getreidemehl, Maismehl, Maisprotein und anderen Maisbestandteilen, Stärke, Kürbismehl, Kartoffelmehl, Roh- und Reststoffe aus der Agrar- und Lebensmittelindustrie, und/ oder Cellulose.

5. Verwendung eines Systems zum Anlocken von Fluginsekten und/oder Spinnentieren in einer gasförmigen Umgebung nach einem der vorherigen Ansprüche zum Locken und gegebenenfalls Töten von Fluginsekten.

6. Verwendung eines Systems zum Anlocken von Fluginsekten und/oder Spinnentieren in einer gasförmigen Umgebung nach Anspruch 5, wobei die Fluginsekten Diptera, Hymenoptera, Coleoptera und Heteroptera sind.

7. Verwendung eines Systems zum Anlocken von Fluginsekten und/oder Spinnentieren in einer gasförmigen Umgebung nach Anspruch 6, wobei die Fluginsekten Fliegen, Mücken, Wanzen oder Termiten sind.

8. Verwendung eines Systems zum Anlocken von Fluginsekten und/oder Spinnentieren in einer gasförmigen Umgebung nach einem der Ansprüche 1 bis 4, zum Locken und gegebenenfalls Töten von Spinnentieren, insbesondere Milben, wie Zecken.

9. Verwendung eines Systems zum Anlocken von Fluginsekten und/oder Spinnentieren in einer gasförmigen Umgebung nach einem der vorherigen Ansprüche, weiterhin enthaltend ein Insektizid und/oder Akarizid, insbesondere eines ausgewählt aus der Gruppe der chemischen Insektizide, chemischen Akarizide, Pflanzenextrakte und/oder entomopathogene Mikroorganismen.

10. Verwendung eines Systems zum Anlocken von Fluginsekten und/oder Spinnentieren in einer gasförmigen Umgebung nach einem der vorherigen Ansprüche, wobei dieses weiterhin als Bestandteil Enzyme, Füllstoffe, Trocknungshilfsmittel, Glukose, Saccharose, weitere Lockstoffe, wie Isolate aus menschlichen Körperschweiss, Vogelfedern, Pferdehaare, Cellulose, Lignin und/oder Quellungshilfen enthält.

11. Verwendung eines Systems zum Anlocken von Fluginsekten und/oder Spinnentieren in einer gasförmigen Umgebung nach einem der vorherigen Ansprüche, wobei die biologisch abbaubaren Biopolymere ausgewählt sind aus Alginat, Carrageenan, Cellulose, Hemicellulose, Stärke, Chitin, Chitosan, Pektinat, Guar Gum, Acacia Gum, poly-D, L-Milchsäure, Gelatine, poly-Aminosäuren, Lignin und Derivaten sowie Mischungen hiervon.

12. Verwendung eines Systems zum Anlocken von Fluginsekten und/oder Spinnentieren in einer gasförmigen Umgebung nach einem der vorherigen Ansprüche, **dadurch gekennzeichnet, dass** dieses in Form von Kapseln, Pellets, Granula, Partikeln, Streifen, Fasern, Beschichtungen bereitgestellt vorliegt.

13. Verwendung eines Systems zum Anlocken von Fluginsekten und/oder Spinnentieren in einer gasförmigen Umgebung nach Anspruch 1, wobei diese Hilfsorganismen Pilze sind, insbesondere B. *bassiana* oder *M. anisopliae* und/oder wobei diese Hilfsorganismen und/oder Enzyme eine Amylase-Aktivität besitzen.

14. Verwendung eines Systems nach einem der Ansprüche 1 bis 13 zum Anlocken und gegebenenfalls Töten von Parasiten-übertragenden Fluginsekten und Spinnentieren.

15. Verwendung eines Systems zum Anlocken von Fluginsekten und/oder Spinnentieren in einer gasförmigen Umgebung nach einem der vorhergehenden Ansprüche, **dadurch gekennzeichnet, dass** dieses ein Immobilisieren, insbesondere Töten, der Fluginsekten und Spinnentiere erlaubt.

## Claims

1. An use of a system for attracting and optionally killing flying insects and/or arachnids in a gaseous environment comprising (i) CO₂-releasing microorganisms, (ii) nutrients specific to these microorganisms, (iii) auxiliary organisms and/or enzymes which support the nutrients supply to the CO₂-releasing microorganisms and (iv) a biodegradable biopolymer, wherein said biodegradable biopolymer embeds or envelopes the further components.

2. The use of a system for attracting flying insects and/or arachnids according to claim 1 for attracting the flying insects and arachnids in a gaseous environment, said system is adapted to release CO₂ over a period more than 20 days.

3. The use of a system for attracting flying insects and/or arachnids according to claim 1 or 2, wherein the CO₂-releasing microorganisms are selected from fungi or bacteria, such as yeast, in particular, baker's yeast.

4. The use of a system for attracting flying insects and/or arachnids in a gaseous environment according to the previous claims, wherein the nutrients are selected from cereal flour, corn flour, corn protein and other corn ingredients, starch, pumpkin flour, potato flour, raw materials and residues from the agricultural and food industry and/or cellulose.

5. The use of a system for attracting flying insects and/or arachnids in a gaseous environment according to any one of the preceding claims for attracting and, optionally, killing flying insects.

6. The use of a system for attracting flying insects and/or arachnids in a gaseous environment according to claim 5, wherein the flying insects are Diptera, Hymenoptera, Coleoptera and Heteroptera.

7. The use of a system for attracting flying insects and/or arachnids in a gaseous environment according to claim 6, wherein the flying insects are flies, mosquitos, bugs or thermites.

8. The use of a system for attracting flying insects and/or arachnids in a gaseous environment according to any one of the claims 1 to 4 for attracting and, optionally, killing arachnids, in particular, mites, such as ticks.

9. The use of a system for attracting flying insects and/or arachnids in a gaseous environment according to any one of the preceding claims, further containing an insecticides and/or acaricide, in particular, selected from the group of chemical insecticides, chemical acaricide, plant extracts and/or entomopathogenic microorganisms.

10. The use of a system for attracting flying insects and/or arachnids in a gaseous environment according to any one of the preceding claims, further containing as constituents enzymes, fillers, drying aids, glucose, sucrose, further attractants, such as isolates from human body sweat, bird feathers, horse hair, cellulose, lignin and/or swelling aids.

11. The use of a system for attracting flying insects and/or arachnids in a gaseous environment according to any one of the preceding claims, wherein the biodegradable biopolymers are selected from alginates, carrageenates, cellulose, hemicellulose, starch, chitin, chitosan, pectinate, guar gum, acacia gum, poly-D, L-lactic acid, gelatine, poly-amino acids, lignin and derivatives and mixtures thereof.

12. The use of a system for attracting flying insects and/or arachnids in a gaseous environment according to any one of the preceding claims, **characterized in, that** it is provided in form of capsules, pellets, granules, particles, stripes, fibres, coatings.

13. The use of a system for attracting flying insects and/or arachnids in a gaseous environment according to claim 1, wherein the auxiliary organisms are fungi, in particular, *B*. *bassiana* or *M. anisopliae* and/or wherein these auxiliary organisms and/or enzymes have an amylase-activity.

14. The use of a system according to any one of claims 1 to 13 for attracting and, optionally, killing parasite transmitting flying insects and arachnids.

15. The use of a system for attracting flying insects and/or arachnids in a gaseous environment according to any one of the preceding claims, **characterized in, that** it allows immobilisation, in particular, killing of flying insects and arachnids.

## Revendications

1. Utilisation d'un système pour piéger et éventuellement tuer des insectes volants et/ou des arachnides dans un environnement gazeux comprenant i) des micro-organismes libérant du CO₂, ii) des nutriments spécifiques pour ces micro-organismes, iii) des organismes auxiliaires et/ou des enzymes, qui soutiennent l'approvisionnement en nutriments des micro-organismes libérant du CO₂, et iv) un biopolymère biodégradable, ce biopolymère biodégradable comprenant ou enfermant les autres composants.

2. Utilisation d'un système pour attirer des insectes volants et/ou des arachnides selon la revendication 1 pour piéger des insectes volants et des arachnides dans un environnement gazeux, ce système étant conçu de telle façon que la libération de CO₂ soit réalisée pendant un temps de plus de 20 jours.

3. Utilisation d'un système pour attirer des insectes volants et/ou des arachnides dans un environnement gazeux selon la revendication 1 ou 2, les micro-organismes libérant du CO₂ étant choisis parmi des champignons et des bactéries, comme des levures, en particulier des levures de boulanger.

4. Utilisation d'un système pour attirer des insectes volants et/ou des arachnides dans un environnement gazeux selon l'une quelconque des revendications précédentes, les nutriments étant choisis parmi de la farine de céréales, de la farine de maïs, des protéines de maïs et d'autres composants du maïs, un amidon, de la farine de courge, de la farine de pomme de terre, des matières premières et des résidus de l'industrie agricole et alimentaire, et/ou des celluloses.

5. Utilisation d'un système pour attirer des insectes volants et/ou des arachnides dans un environnement gazeux selon l'une quelconque des revendications précédentes pour piéger et éventuellement tuer des insectes volants.

6. Utilisation d'un système pour attirer des insectes volants et/ou des arachnides dans un environnement gazeux selon la revendication 5, les insectes volants étant diptères, des hyménoptères, des coléoptères et des hétéroptères.

7. Utilisation d'un système pour attirer des insectes volants et/ou des arachnides dans un environnement gazeux selon la revendication 6, les insectes volants étant des mouches, des moustiques, des punaises ou des termites.

8. Utilisation d'un système pour attirer des insectes volants et/ou des arachnides dans un environnement gazeux selon l'une quelconque des revendications 1 à 4, pour piéger et éventuellement tuer des arachnides, en particulier des acariens, comme des tiques.

9. Utilisation d'un système pour attirer des insectes volants et/ou des arachnides dans un environnement gazeux selon l'une quelconque des revendications précédentes, contenant en outre un insecticide et/ou un acaricide, en particulier choisi dans le groupe des insecticides chimiques, des acaricides chimiques, des extraits végétaux et/ou des micro-organismes entomopathogènes.

10. Utilisation d'un système pour attirer des insectes volants et/ou des arachnides dans un environnement gazeux selon l'une quelconque des revendications précédentes, celui-ci contenant en outre en tant que composant des enzymes, des charges, des auxiliaires de séchage, du glucose, du saccharose, d'autres substances de piégeage, comme des isolats provenant de la sueur corporelle humaine, des plumes d'oiseaux, des crins de cheval, de la cellulose, de la lignine et/ou des auxiliaires de gonflement.

11. Utilisation d'un système pour attirer des insectes volants et/ou des arachnides dans un environnement gazeux selon l'une quelconque des revendications précédentes, les biopolymères biodégradables étant choisis parmi un alginate, un carraghénane, une cellulose, une hémicellulose, un amidon, une chitine, un chitosane, un pectinate, une gomme de guar, une gomme arabique, un poly(acide D,L-lactique), une gélatine, des poly(acide aminé), une lignine et des dérivés ainsi que des mélanges correspondants.

12. Utilisation d'un système pour attirer des insectes volants et/ou des arachnides dans un environnement gazeux selon l'une quelconque des revendications précédentes, **caractérisée en ce que** celui-ci se trouve sous forme de capsules, de pastilles, de granulés, de particules, de bandes, de fibres, de revêtements.

13. Utilisation d'un système pour attirer des insectes volants et/ou des arachnides dans un environnement gazeux selon la revendication 1, ces organismes auxiliaires étant des champignons, en particulier *B. bassiana* ou *M. anisopliae* et/ou ces organismes auxiliaires et/ou ces enzymes possédant une activité d'amylase.

14. Utilisation d'un système selon l'une quelconque des revendications 1 à 13 pour attirer et éventuellement tuer des insectes volants et des arachnides transmetteurs de parasites.

15. Utilisation d'un système pour attirer des insectes volants et/ou des arachnides dans un environnement gazeux selon l'une quelconque des revendications précédentes, **caractérisée en ce que** celui-ci permet une immobilisation, en particulier une destruction, des insectes volants et des arachnides.
